# FASCICULE DE BREVET EUROPEEN

(11) **EP 0 967 207 B1**
(45) Date de publication et mention de la délivrance du brevet: **11.09.2002**
(21) Numéro de dépôt: 99401565.9
(22) Date de dépôt: 24.06.1999
(51) Int. Cl.: C07D 233/64, C07D 401/04, C07D 405/04, C07D 409/04, C07D 403/04, C07D 417/04, A61K 31/415

(54) **Nouveaux dérivés de nitrone, leur procédé de préparation et les compositions pharmaceutiques qui les contiennent**
Nitronderivate, Verfahren zu deren Herstellung und sie enthaltende pharmazeutische Zusammensetzungen
Nitrone derivatives, process for their preparation and pharmaceutical compositions containing them

(30) Priorité: 26.06.1998 FR 9808116
(43) Date de publication de la demande: 29.12.1999
(73) Titulaire: LES LABORATOIRES SERVIER, 92200 Neuilly sur Seine (FR)
(72) Inventeur: Goldstein, Solo, 92150 Suresnes (FR); Dhainaut, Alain, 78400 Chatou (FR); Tizot, André, 91370 Verrières-le-Buisson (FR); Lockhart, Brian, 78290 Croissy sur Seine (FR); Lestage, Pierre, 78170 La-Celle-Saint-Cloud (FR)

(56) Documents cités:
- WO-A-92/22290
- WO-A-95/11227
- WO-A-97/19054

## Description

La présente invention concerne de nouveaux dérivés de nitrone, leur procédé de préparation et les compositions pharmaceutiques qui les contiennent.

Ces nouveaux composés constituent des antioxydants, piégeurs d'espèces oxygénées réactives, capables de s'opposer à des "stress oxydants" au niveau cérébral.

Selon la théorie radicalaire du vieillissement de Hartman, les agressions oxydantes successives créent des conditions de "stress oxydant", c'est-à-dire un déséquilibre entre les systèmes protecteurs en faveur des pro-oxydants.

Ces agressions conduisent à de nombreuses modifications moléculaires, notamment des lipides membranaires polyinsaturés, des protéines et des acides nucléiques. Les organismes, humain et animal, disposent de différents mécanismes de défense agissant en synergie. Ces mécanismes sont de nature enzymatique (superoxyde dismutase, catalase, glutathion peroxydase) ou non enzymatique (comme les vitamines E ou C qui permettent physiologiquement de contrôler l'action des radicaux libres). Mais, avec l'âge, cette protection devient moins efficace, voire inefficace, en particulier du fait de l'inaction oxydative de nombreuses enzymes dont celles participant à ces mécanismes de défense. Ainsi, dans certaines affections liées à la sénescence, telles que l'athérosclérose, la cataracte, le diabète non insulino-dépendant, le cancer ou les maladies neurodégénératives chroniques, de nombreux travaux ont pu montrer qu'elles étaient associées à ces conditions de "stress oxydant".

Le système nerveux central est particulièrement sensible au "stress oxydant" en raison de sa haute consommation d'oxygène, des niveaux relativement faibles de ses défenses antioxydantes, et de la forte teneur en fer de certaines aires cérébrales. Ceci explique que le "stress oxydant" puisse être l'un des facteurs étiologiques principaux du vieillissement cérébral, ainsi que des maladies neurodégénératives chroniques, particulièrement la maladie d'Alzheimer et les neurodégénérescences des ganglions de la base.

Différents piégeurs de radicaux libres intervenant comme agents antioxydants ont été décrits dans la littérature. C'est le cas plus particulièrement des composés décrits dans la demande de brevet WO 92/22290.

Les composés de la présente invention, outre le fait qu'ils soient nouveaux, se sont révélés être des antioxydants plus puissants que ceux décrits dans la littérature et non toxiques. Des calculs théoriques HOMO/LUMO de haut niveau mettent en évidence les particularités de ces produits en ce qui concerne leur efficacité de piéger des radicaux libres tant carbonés (LUMO basse) qu'oxygénés (HOMO haute). Ces propriétés caractéristiques rendent donc les composés de l'invention potentiellement utiles pour le traitement et la prévention des pathologies, d'une part liées au vieillissement et en particulier au vieillissement cérébral, et d'autre part dues au stress oxydant.

Les composés de l'invention constituent ainsi des agents thérapeutiques, utiles en tant qu'agents antioxydants, et capables de s'opposer aux troubles cognitifs associés au vieillissement cérébral ou aux maladies neurodégénératives, ainsi qu'à la mort neuronale associée non seulement aux maladies neurodégénératives aiguës comme par exemple l'ischémie et l'épilepsie, mais encore à celle associée aux maladies neurodégénératives progressives, comme par exemple la maladie d'Alzheimer, la maladie de Pick ou les neurodégénérescences des ganglions de la base.

Plus spécifiquement, les composés de la présente invention concernent les composés de formule (I) : 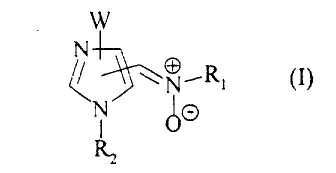 dans laquelle
- **W**: représente un groupement aryle, arylalkyle (C₁-C₆) linéaire ou ramifié, hétéroaryle ou hétéroarylalkyle (C₁-C₆) linéaire ou ramifié,
- **R**_{**1**}: représente un groupement alkyle (C₁-C₆) linéaire ou ramifié, alkényle (C₂-C₆) linéaire
ou ramifié, alkynyle (C₂-C₆) linéaire ou ramifié, aryle, cycloalkyle, arylalkyle (C₁-C₆) linéaire ou ramifié, ou cycloalkylalkyle (C₁-C₆) linéaire ou ramifié,
- **R**_{**2**}: représente un atome d'hydrogène, ou un groupement alkyle (C₁-C₆) linéaire ou ramifié,
leurs isomères optiques, ainsi que leurs sels d'addition à un acide ou à une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique, etc...

Parmi les bases pharmaceutiquement acceptables, on peut citer à titre non limitatif, l'hydroxyde de sodium, l'hydroxyde de potassium, la triéthylamine, la tertbutylamine, etc...

Par cycloalkyle, on entend un groupement mono ou bicyclique, saturé ou insaturé mais non aromatique, comportant de 3 à 10 atomes de carbone, chacun de ces groupements pouvant être substitué de façon identique ou différente, par un ou plusieurs atomes d'halogéne, groupements hydroxy, alkyle (C₁-C₆) linéaire ou ramifié, alkoxy (C₁-C₆) linéaire ou ramifié, acyle (C₁-C₆) linéaire ou ramifié, amino (lui-même éventuellement substitué par un ou deux groupements, identiques ou différents, alkyle (C₁-C₆) linéaire ou ramifié), alkoxycarbonyle (C₁-C₆) linéaire ou ramifié, mercapto, alkylthio (C₂-C₆) linéaire ou ramifié.

Par groupement hétérocycloalkyle, on entend un groupement cycloalkyle dans lequel un, deux ou trois atomes de carbone sont remplacés par un hétéroatome, choisi de façon identique ou différente, parmi azote, oxygène et soufre.

Par groupement aryle, on entend un groupement phényle, naphtyle, indényle, tétrahydronaphtyle, dihydronaphtyle, dihydroindényle, chacun de ces groupements étant éventuellement substitué de façon identique ou différente, par un ou plusieurs groupements Q, tel que Q représente un atome d'halogène, un groupement hydroxy, cyano, nitro, alkyle (C₁-C₆) linéaire ou ramifié, aryle, hétéroaryle (étant entendu que dans le cas où Q représente un groupement aryle ou hétéroaryle, alors ledit groupement aryle ou ledit groupement hétéroaryle ne peut pas être substitué par un autre groupement aryle ou hétéroaryle), alkoxy (C₁-C₆) linéaire ou ramifié, trihalogénoalkyle (C₁-C₆) linéaire ou ramifié, trihalogénoalkoxy (C₁-C₆) linéaire ou ramifié, acyle (C₁-C₆) linéaire ou ramifié, carboxy, alkoxycarbonyle (C₁-C₆) linéaire ou ramifié, alkylcarbonyloxy (C₁-C₆) linéaire ou ramifié, amino (substitué éventuellement par un ou deux groupements, identiques ou différents, choisis parmi alkyle (C₁-C₆) linéaire ou ramifié, hydroxyalkyle (C₁-C₆) linéaire ou ramifié, alkoxyalkyle (C₁-C₆) linéaire ou ramifié, aminoalkyle (C₁-C₆) linéaire ou ramifié, et mono ou dialkylaminoalkyle (C₁-C₆) linéaire ou ramifié), mercapto, alkylthio (C₁-C₆) linéaire ou ramifié, aminocarbonyle (la partie amino étant éventuellement substituée par un ou deux groupements identiques ou différents, alkyle (C₁-C₆) linéaire ou ramifié), alkylcarbonylamino (C₁-C₆) linéaire ou ramifié, hydroxysulfonyle, alkylsulfonyle (C₁-C₆) linéaire ou ramifié, ou arylsulfonyle.

Par hétéroaryle, on entend un groupement aryle, mono ou bicyclique, de 5 à 12 chaînons contenant un, deux ou trois hétéroatomes, identiques ou différents, choisis parmi oxygène, azote ou soufre, étant entendu que l'hétéroaryle peut être éventuellement substitué, de façon identique ou différente, par un ou plusieurs groupements Q tels que définis précédemment, et que dans le cas où l'hétéroaryle est bicyclique, alors un des cycles peut être partiellement hydrogéné.

Selon une variante particulièrement avantageuse, les composés préférés de l'invention sont ceux pour lesquels le groupement W est lié en position 2 sur le cycle imidazolique.

Selon une autre variante de l'invention, les composés préférés sont ceux pour lesquels le groupement nitrone substitué 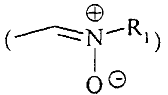 est lié en position 2 sur le cycle imidazolique.

Les substituants R₁ préférés selon l'invention sont les groupements alkyle (C₁-C₆) linéaire ou ramifié. D'une façon particulièrement avantageuse, le substituant R₁ préféré est le groupement tertio-butyle.

Les substituants R₂ préférés selon l'invention sont l'atome d'hydrogène et le groupement alkyle (C₁-C₆) linéaire ou ramifié.

Les substituants W préférés selon l'invention sont les groupements aryles ou les groupements hétéroaryles et plus préférentiellement les groupements phényles substitués ou non, naphtyles substitués ou non, pyridinyles substitués ou non, furyles substitués ou non, thiényles substitués ou non, pyrrolyles substitués ou non, imidazolyles substitués ou non, thiazolyles substitués ou non, quinolyles substitués ou non, ou indolyles substitués ou non.

Le composé préféré selon l'invention est le (Z)-α-(2-phényl-1*H*-imidazol-4-yl)-N-tertbutylnitrone.

Les isomères optiques ainsi que les sels d'addition à un acide ou à une base pharmaceutiquement acceptable des composés préférés font partie intégrante de l'invention.

L'invention s'étend également au procédé de préparation des composés de formule (I) caractérisé en ce qu'on utilise comme produit de départ un composé de formule (II) 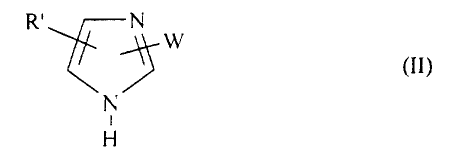 dans laquelle W est tel que défini dans la formule (I) et R' représente un atome d'hydrogène ou un groupement trifluorométhyle,
- composé de formule (II), dans le cas où W est lié en position 4 sur le cycle imidazolique et R' représente un atome d'hydrogène, que l'on traite par de l'ortho-formiate d'éthyle en présence d'acide para-toluène sulfonique, pour conduire aux composés de formule (III) : 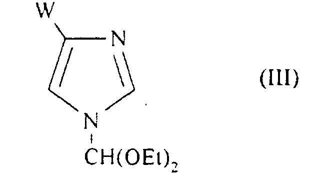 dans laquelle W est tel que défini dans la formule (I),
   composés de formule (III), que l'on traite en présence d'une base forte par du diméthylformamide, suivie d'une hydrolyse acide, pour conduire aux composés de formule (IV): 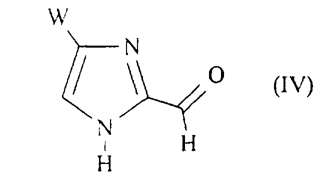 dans laquelle W est tel que défini dans la formule (1),
   composé de formule (IV)
   * que l'on traite en par une hydroxylamine substituée de formule (V) :

      HO-NH-R₁ (V)

      dans laquelle R₁ est tel que défini dans la formule (I),
      pour conduire aux composés de formule (I/a), cas particulier des composés de formule (I) : 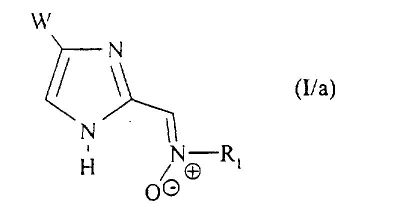 dans laquelle W et R₁ sont tels que définis dans la formule (I),
   * ou que l'on traite, si on le souhaite, avec un réactif électrophile selon des conditions classiques de synthèse organique, pour conduire aux composés de formules (IV/A) et (IV/B) : 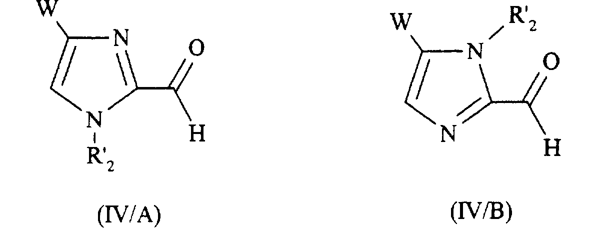 dans lesquelles W est tel que défini dans la formule (I) et R'₂ a la même définition que R₂ dans la formule (I), excepté que R'₂ ne peut pas représenter un atome d'hydrogène,
   composés de formules (IV/A) et (IV/B) que l'on sépare et/ou que l'on purifie si on le souhaite selon des méthodes classiques de séparation et de purification, puis que l'on soumet à l'action d'un composé de formule (V) tel que défini précédemment, pour conduire, respectivement, aux composés de formules (I/b) et (I/c), cas particuliers des composés de formule (I) : 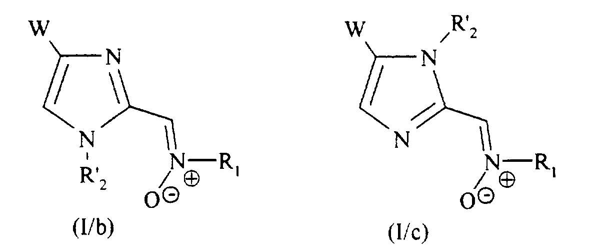 dans lesquelles R₁, R'₂ et W sont tels que définis précédemment,
- ou composé de formule (II), dans le cas où W est lié en position 2 sur le cycle imidazolique et R' représente un groupement trifluorométhyle : 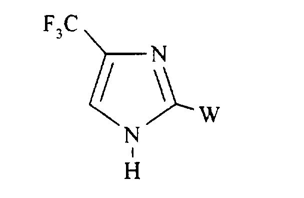 que l'on traite soit par de l'hydroxyde d'ammonium, suivi d'une hydrolyse, soit par de la soude suivi d'une étape d'addition de N-méthoxy-N-méthylamine puis d'une réaction classique de réduction,
   pour conduire aux composés de formule (VI) : 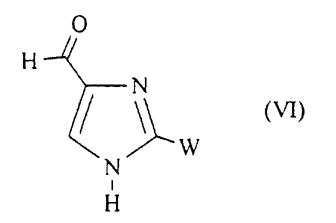 dans laquelle W est tel que défini dans la formule (I),
   composés de formule (VI) que l'on soumet à l'action d'un composé de formule (V) tel que défini précédemment, pour conduire aux composés de formule (I/d), cas particulier des composés de formule (I) 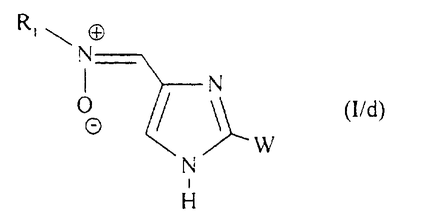 dans laquelle R₁ et W sont tels que définis dans la formule (I),
   ou composés de formule (VI), que l'on traite, si on le souhaite, avec un réactif électrophile, selon des conditions classiques de synthèse organique, pour conduire aux composés de formules (VI/A) et (VI/B) 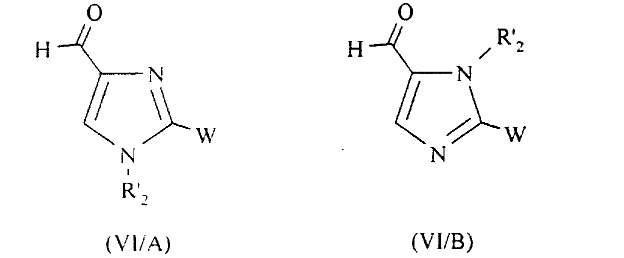 dans lesquelles W et R'₂ sont tels que définis précédemment,
   composés de formules (VI/A) et (VI/B) que l'on sépare et/ou que l'on purifie selon des méthodes classiques de séparation et de purification, puis que l'on soumet à l'action d'un composé de formule (V), pour conduire, respectivement, aux composés de formules (I/e) et (I/f), cas particulier des composés de formule (I) : 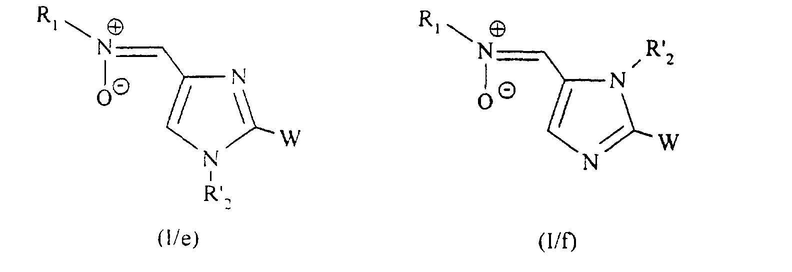 dans lesquelles R₁, R'₂ et W sont tels que définis précédemment,
les composés (I/a) à (I/f) formant l'ensemble des composés de l'invention, que l'on purifie, le cas échéant, selon une technique classique de purification, qui peuvent, si on le désire, être séparés en leurs différents isomères selon une technique classique de séparation, et que l'on transforme, le cas échéant, en leurs sels d'addition à un acide ou à une base pharmaceutiquement acceptable

Les composés de formule (II) et (IV) sont soit des composés commerciaux, soit obtenus selon des méthodes connues de la synthèse organique comme celles décrites dans les articles *J. Med. Chem.* 1975, 18, 895. *J. Org. Chem.* 1986, 51, 3228 et *Tetrahedron. Lett*., 1981, 3815.

La présente invention a également pour objet les compositions pharmaceutiques renfermant comme principe actif au moins un composé de formule (I), ses isomères optiques ou un de ses sels d'addition à un acide ou à une base pharmaceutiquement acceptable, seul ou en combinaison avec un ou plusieurs excipients ou véhicules inertes non toxiques, pharmaceutiquement acceptables

Parmi les compositions pharmaceutiques selon l'invention, il sera cité plus particulièrement celles qui conviennent pour l'administration orale, parentérale (intraveineuse, intramusculaire ou sous-cutanée), per ou transcutanée, nasale, rectale, perlinguale, oculaire ou respiratoire, et notamment les comprimés simples ou dragéifiés, les comprimés sublinguaux, les sachets, les gélules, les tablettes, les suppositoires, les crèmes, les pommades, les gels dermiques, les préparations injectables ou buvables, les aérosols, les gouttes oculaires ou nasales, etc .

La posologie utile varie selon l'âge et le poids du patient, la voie d'administration, la nature et la sévérité de l'affection, et la prise de traitements éventuels et s'échelonne de 10 mg à 500 mg en une ou plusieurs prises par jour.

Les exemples suivants illustrent l'invention mais ne la limitent en aucune façon.

Les produits de départ utilisés sont des produits connus ou préparés selon des modes opératoires connus

### Exemple 1 : (Z)-α-(2-Phényl-1H-imidazol-4-yl)-N-tertbutylnitrone

### Stade A : 2-phényl-4-trifluorométhyl-1H-imidazole

On procède selon les conditions opératoires décrites dans J. Med. Chem., 1975, 18, 895-900, en utilisant comme substrat le benzaldéhyde.

### Stade B : 2-phényl-4-cyano-1H-imidazole

Le composé obtenu dans le stade A est soumis au protocole expérimental décrit dans J. Org. Chem , 1986, 51, 3228-3231

### Stade C : 2-phényl-1H-4-imidazolcarbaldéhyle

Une solution contenant 1,6 mmol du composé obtenu dans le stade B dans 3 ml de tétrahydrofurane anhydre est placée à -78°C, sous atmosphère d'argon, et 2,45 ml d'hydrure de diisobutylaluminium (1,1 M dans le tétrahydrofurane) sont additionnés. Après 2,5 heures, 2,45 ml de réactif sont à nouveau ajoutés et après 1 heure de réaction, la température est ramenée à -45°C. 1 ml de méthanol est alors additionné au milieu réactionnel. Après 20 minutes d'agitation, la température est remontée à -20°C et 3 ml d'HCI 1N sont additionnés. L'agitation est poursuivie 30 minutes en laissant revenir le milieu réactionnel à température ambiante. La solution est alors diluée avec du dichlorométhane puis extraite à l'eau. La phase organique est ensuite séchée sur sulfate de magnésium puis concentrée sous pression réduite permettant de recueillir un précipité Celui-ci est filtré, rincé au dichlorométhane, séché, permettant d'isoler le produit attendu.

### Stade D : (Z)-α-(2-phényl-1H-imidazol-4-yl)-N-tertbutylnitrone

0,05 mol du composé obtenu dans le stade C, 0,07 mol de chlorhydrate de N-terbutylhydroxylamine et 6 g d'hydrogénocarbonate de sodium en solution dans 50 ml d'éthanol sont agités à 60°C sous atmosphère d'argon pendant 20 heures. Le milieu réactionnel est ensuite ramené à température ambiante, et dilué avec 300 ml de dichlorométhane. La phase organique est extraite à l'eau, séchée sur sulfate de magnésium puis concentrée sous pression réduite. L'huile résiduelle, reprise rapidement dans l'éther éthylique, cristallise Après filtration et séchage sous vide, on isole le produit attendu.
*Point de fusion : 209-210°C*

| *Microanalyse élémentaire :* | | | |
|---|---|---|---|
| | *C* | *H* | *N* |
| *% Calculé* | *69,11* | *7,04* | *17,27* |
| *% Trouvé* | *68,85* | *7,04* | *17,02* |

### Exemple 2 : (Z)-α-[2-(4-méthylphényl)-1H-imidazol-4-yl]-N-tertbutylnitrone

On procède comme dans l'exemple 1, des stades A à D, en utilisant comme substrat au stade A le 4-méthyl-benzaldéhyde
*Point de fusion : 174-176°C*

| *Microanalyse élémentaire :* | | | |
|---|---|---|---|
| | *C* | *H* | *N* |
| *% Calculé* | *70,01* | *7,44* | *16,33* |
| *% Trouvé* | *69,69* | *7,57* | *16,03* |

### Exemple 3 : (Z)-α-[2-(4-méthoxyphényl)-1H-imidazol-4-yl]-N-tertbutylnitrone

### Préparation A : 4-carboxy-2-(4-méthoxyphényl)-1H-imidazole

Une solution de 3,8 g de 2-(4-méthoxyphényl)-4-trifluorométhylimidazole dans 40 ml de soude 2N est chauffée sous agitation à 90°C pendant 1 heure. Après retour à température ambiante, la solution est amenée à pH 6 avec de l'acide chlorhydrique 2N. Le précipité est filtré, rincé à l'eau et séché pour donner le produit attendu.

### Stade A1 : 4-(N-méthyl-N-méthoxyaminocarbonyl)-2-(4-méthoxyphényl)-1H-imidazole

A une solution contenant 13 mmol du produit obtenu dans la préparation A dans 130 ml de dichlorométhane sont additionnées 15 mmol de tétrafluoroborate de O-benzotriazole-1-yl-N,N,N',N'-tétraméthyluronium, 49 mmol de N,N-diisopropyléthylamine et 15 mmol de N-méthoxyméthylamine Après 20 heures de réaction à température ambiante, le milieu réactionnel est lavé à l'eau La phase organique est ensuite séchée sur sulfate de magnésium, filtrée et concentrée sous pression réduite Le résidu est alors trituré dans l'éther et le solide obtenu est filtré puis séché, permettant d'isoler le produit attendu.

### Stade B1 : 2-(4-méthoxyphényl)-1H-imidazol-4-carbaldéhyde

A une solution contenant 6,1 mmol du produit obtenu dans le stade A1 dans 50 ml de tétrahydrofurane est ajouté, sous atmosphère d'argon, 0,5 g d'hydrure d'aluminium lithium. Après une heure de réaction à -10°C, 0.2 g d'hydrure d'aluminium lithium est additionné et l'agitation est maintenue une heure à 0°C La réaction est ensuite hydrolysée par addition d'eau puis par une solution de soude 4N Après filtration du milieu réactionnel, la solution est concentrée sous pression réduite Une chromatographie sur gel de silice (dichlorométhane/méthanol 9/1) permet d'isoler le produit attendu.

### Stade C1 : (Z)-α-[2-(4-méthoxyphényl)-1H-imidazol-4-yl]-N-tertbutylnitrone

On procède comme dans le stade D de l'exemple 1, en utilisant comme substrat le produit obtenu dans le stade B1.
*Point de fusion : 198-201°C*

| *Microanalyse élémentaire :* | | | |
|---|---|---|---|
| | *C* | *H* | *N* |
| *% Calculé* | *65,91* | *7,01* | *15,37* |
| *% Trouvé* | *65,65* | *7,30* | *14,91* |

### Exemple 4 : (Z)-α-[2-(4-chlorophényl)-1H-imidazol-4-yl]-N-tertbutylnitrone

On procède comme dans l'exemple 1, des stades A à D, en utilisant comme substrat au stade A le 4-chlorobenzaldéhyde.
*Point de fusion : 212-215°C*

### Exemple 5 : (Z)-α-[2-(4-pyridinyl)-1H-imidazol-4-yl]-N-tertbutylnitrone

On procède comme dans l'exemple 1, des stades A à D, en utilisant comme substrat au stade A, le 4-pyridinecarbaldéhyde
*Point de fusion : 239-241°C*

| *Microanalyse élémentaire :* | | | |
|---|---|---|---|
| | *C* | *H* | *N* |
| *% Calculé* | *63,92* | *6,60* | *22,93* |
| *% Trouvé* | *63,94* | *6,58* | *23,03* |

### Exemple 6 : (Z)-α-[2-(2-naphtyl)-1H-imidazol-4-yl]-N-tertbutylnitrone

On procède comme dans l'exemple 1, des stades A à D, en utilisant comme substrat au stade A, le 2-naphthaldéhyde
*Point de fusion : 200-202°C*

| *Microanalyse élémentaire :* | | | |
|---|---|---|---|
| | *C* | *H* | *N* |
| *% Calculé* | *73,70* | *6,53* | *14,32* |
| *% Trouvé* | *73,69* | *6,79* | *14,30* |

### Exemple 7 : (Z)-α-[2-(2-furyl)-1H-imidazol-4-yl]-N-tertbutylnitrone

On procède comme dans l'exemple 1, des stades A à D, en utilisant comme substrat au stade A, le 2-furylcarbaldéhyde
*Point de fusion : 103°C (décomposition)*

### Exemple 8 : (Z)-α-[1-méthyl-2-phényl-1H-imidazol-4-yl]-N-tertbutylnitrone

### Stade E : 1-méthyl-2-phényl-4-trifluorométhyl-1H-imidazole

A une solution de 10 mmol du composé obtenu dans le stade A de l'exemple 1, dans 15 ml de toluène, sont additionnés, à température ambiante, 10 mmol de NaH, puis après 15 minutes, 10 mmol d'iodure de méthyle diluées dans 3 ml de toluène. Après 2 heures, la réaction est concentrée sous pression réduite permettant d'obtenir une huile qui précipite par addition d'eau Une filtration permet d'isoler le produit attendu.

### Stade F : (Z)-α-[1-méthyl-2-phényl-1H-imidazole-4-yl]-N-tertbutylnitrone

On procède comme dans l'exemple 1, des stades B à D, en utilisant dans le stade B, le produit obtenu dans le stade E

### Exemple 9 : (Z)-α-[1-Méthyl-2-phényl-1H-imidazol-5-yl]-N-tertbutylnitrone

Le produit est obtenu en tant que co-produit lors de la synthèse du composé de l'exemple 8.
*Point de fusion : 155-157°C*

| *Microanalyse élémentaire :* | | | |
|---|---|---|---|
| | *C* | *H* | *N* |
| *% Calculé* | *70,01* | *7,44* | *16,33* |
| *% Trouvé* | *70,21* | *7,47* | *16,40* |

### Exemple 10 : (Z)-α-[2-(3-Thiényl)-1H-imidazol-4-yl]-N-tertbutylnitrone

On procède comme dans l'exemple 1, des stades A à D, en utilisant comme substrat au stade A, le 3-thiénylcarbaldéhyde
*Point de fusion : 197-198°C*

| *Microanalyse élémentaire :* | | | | |
|---|---|---|---|---|
| | *C* | *H* | *N* | *S* |
| *% Calculé* | *57,81* | *6,06* | *16,85* | *12,86* |
| *% Trouvé* | *57,85* | *6,07* | *16,42* | *13,02* |

### Exemple 11 : (Z)-α-[2-(3-Pyridinyl)-1H-imidazol-4-yl]-N-tertbutylnitrone

On procède comme dans l'exemple 1, des stades A à D, en utilisant comme substrat au stade A, la nicotinealdéhyde
*Point de fusion : 174-176°C*

| *Microanalyse élémentaire :* | | | |
|---|---|---|---|
| | *C* | *H* | *N* |
| *% Calculé* | *63,92* | *6,60* | *22,93* |
| *% Trouvé* | *63,27* | *6,67* | *22,24* |

### Exemple 12 : (Z)-α-[2-(2-Pyridinyl)-1H-imidazol-4-yl]-N-tertbutylnitrone

On procède comme dans l'exemple 1, des stades A à D, en utilisant comme substrat au stade A, la 2-pyridinecarbaldéhyde
*Point de fusion : 172-173°C*

| *Microanalyse élémentaire :* | | | |
|---|---|---|---|
| | *C* | *H* | *N* |
| *%Calculé* | *63,92* | *6,60* | *22,93* |
| *% Trouvé* | *63,74* | *6,65* | *22,83* |

### Exemple 13 : (Z)-α-[2-(3-Furyl)-1H-imidazol-4-yl]-N-tertbutylnitrone

On procède comme dans l'exemple 1, des stades A à D, en utilisant comme substrat au stade A, le 3-furylcarbaldéhyde
*Point de fusion : 160°C*

### Exemple 14 : (Z)-α-[2-(4-Trifluorométhylphényl)-1H-imidazol-4-yl]-N-tertbutylnitrone

On procède comme dans l'exemple 1, des stades A à D, en utilisant comme substrat au stade A, le 4-(trifluorométhyl)benzaldéhyde
*Point de fusion : 226°C*

| *Microanalyse élémentaire :* | | | |
|---|---|---|---|
| | *C* | *H* | *N* |
| *% Calculé* | *57,87* | *5,18* | *13,50* |
| *% Trouvé* | *57,88* | *5,12* | *13,50* |

### Exemple 15 : (Z)-α-[2-(1-Naphtyl)-1H-imidazol-4-yl]-N-tertbutylnitrone

On procède comme dans l'exemple 1, des stades A à D, en utilisant comme substrat au stade A, le 1-naphtylcarbaldéhyde
*Point de fusion : 95°C (décomposition)*

### Exemple 16 : (Z)-α-[2-(1H-Imidazol-4-yl)-1H-imidazol-4-yl]-N-tertbutylnitrone

On procède comme dans l'exemple 1, des stades A à D, en utilisant comme substrat au stade A, le 1*H*-imidazol-4-carbaldéhyde
*Point de fusion : 220°C*

### Exemple 17 : (Z)-α-[2-(4-Fluorophényl)-1H-imidazol-4-yl]-N-tertbutylnitrone

On procède comme dans l'exemple 1, des stades A à D, en utilisant comme substrat au stade A, le 4-(fluoro)-benzaldéhyde
*Point de fusion : 192-194°C*

| *Microanalyse élémentaire :* | | | |
|---|---|---|---|
| | *C* | *H* | *N* |
| *% Calculé* | *64,35* | *6,17* | *16,08* |
| *% Trouvé* | *64,49* | *6,15* | *15,88* |

### Exemple 18 : (Z)-α-[2-(2-Chlorophényl)-1H-imidazol-4-yl]-N-tertbutylnitrone

On procède comme dans l'exemple 1, des stades A à D, en utilisant comme substrat au stade A, le 2-(chloro)-benzaldéhyde
*Point de fusion : 132-134°C*

| *Microanalyse élémentaire :* | | | | |
|---|---|---|---|---|
| | *C* | *H* | *N* | *Cl* |
| *% Calculé* | *60,54* | *5,81* | *15,13* | *12,76* |
| *% Trouvé* | *60,63* | *5,72* | *15,01* | *12,77* |

### Exemple 19 : (Z)-α-[2-(3-Chlorophényl)-1H-imidazol-4-yl]-N-tertbutylnitrone

On procède comme dans l'exemple 1, des stades A à D, en utilisant comme substrat au stade A, le 3-(chloro)-benzaldéhyde
*Point de fusion : 181-183°C*

| *Microanalyse élémentaire :* | | | | |
|---|---|---|---|---|
| | *C* | *H* | *N* | *Cl* |
| *% Calculé* | *60,54* | *5,81* | *15,13* | *12,76* |
| *% Trouvé* | *60,52* | *5,91* | *14,96* | *12,90* |

### Exemple 20 : (Z)-α-[2-(4-Nitrophényl)-1H-imidazol-4-yl]-N-tertbutylnitrone

On procède comme dans l'exemple 1, des stades A à D, en utilisant comme substrat au stade A, le 4-(nitro)-benzaldéhyde.
*Point de fusion : 253°C*

| *Microanalyse élémentaire :* | | | |
|---|---|---|---|
| | *C* | *H* | *N* |
| *% Calculé* | *58,33* | *5,59* | *19,43* |
| *% Trouvé* | *57,95* | *5,51* | *18,99* |

### Exemple 21 : (Z)-α-[2-(4-Diméthylaminophényl)-1H-imidazol-4-yl]-N-tertbutylnitrone

On procède comme dans l'exemple 1, des stades A à D, en utilisant comme substrat au stade A, le 4-(diméthylamino)-benzaldéhyde.
*Point de fusion : 190-192°C*

| *Microanalyse élémentaire :* | | | |
|---|---|---|---|
| | *C* | *H* | *N* |
| *% Calculé* | *67,11* | *7,74* | *19,56* |
| *% Trouvé* | *66,90* | *7,78* | *19,30* |

### Exemple 22 : (Z)-α-[2-(2-Thiazolyl)-1H-imidazol-4-yl]-N-tertbutylnitrone

On procède comme dans l'exemple 1, des stades A à D, en utilisant comme substrat au stade A, le 2-(thiazolyl)-carbaldéhyde.
*Point de fusion : 170°C*

### Exemple 23 : (Z)-α-[2-(3,4-Diméthoxyphényl)-1H-imidazol-4-yl]-N-tertbutylnitrone

On procède comme dans l'exemple 1, des stades A à D, en utilisant comme substrat au stade A, le (3,4-diméthoxy)-benzaldéhyde.
*Point de fusion : 202-205°C*

### Exemple 24 : (Z)-α-[2-(3,4-Méthylènedioxyphényl)-1H-imidazol-4-yl]-N-tertbutylnitrone

On procède comme dans l'exemple 1, des stades A à D, en utilisant comme substrat au stade A, le (3,4-méthylènedioxy)benzaldéhyde.

### Exemple 25 : (Z)-α-(2-Phénéthyl-1H-imidazol-4-yl)-N-tertbutylnitrone

On procède comme dans l'exemple 1, des stades A à D, en utilisant comme substrat au stade A, le 3-phénylpropionaldéhyde

### Exemple 26 : (Z)-α-[2-(5-Nitro-3-thiényl)-1H-imidazol-4-yl]-N-tertbutylnitrone

On procède comme dans l'exemple 1, des stades A à D, en utilisant comme substrat au stade A, le 2-nitrothiophène-4-carbaldéhyde.

### Exemple 27 : (Z)-α-[2-(5-Méthyl-2-thiényl)-1H-imidazol-4-yl]-N-tertbutylnitrone

On procède comme dans l'exemple 1, des stades A à D, en utilisant comme substrat au stade A, la 5-méthylthiophène-2-carbaldéhyde

### Exemple 28 : (Z)-α-[2-(4-Méthylthiophényl)-1H-imidazol-4-yl]-N-tertbutylnitrone

On procède comme dans l'exemple 1, des stades A à D, en utilisant comme substrat au stade A, le 4-(méthylthio)-benzaldéhyde

### Exemple 29 : (Z)-α-[2-(1-Méthyl-1H-pyrrol-2-yl)-1H-imidazol-4-yl]-N-tertbutylnitrone

On procède comme dans l'exemple 1, des stades A à D, en utilisant comme substrat au stade A, le 1-méthylpyrrole-2-carbaldéhyde

### Exemple 30 : (Z)-α-[2-(1-Méthyl-1H-indol-3-yl)-1H-imidazol-4-yl]-N-tertbutylnitrone

On procède comme dans l'exemple 1, des stades A à D, en utilisant comme substrat au stade A, le 1-méthylindole-3-carbaldéhyde.

### Exemple 31 : (Z)-α-[2-(3-Méthyl-benzo[b]thiophène-2-yl)-1H-imidazol-4-yl]-N-tertbutylnitrone

On procède comme dans l'exemple 1, des stades A à D, en utilisant comme substrat au stade A, le 3-méthylbenzo[b]thiophène-2-carbaldéhyde

### Exemple 32 : (Z)-α-[2-(4-Méthoxy-1-naphtyl)-1H-imidazol-4-yl]-N-tertbutylnitrone

On procède comme dans l'exemple 1, des stades A à D, en utilisant comme substrat au stade A, le 4-méthoxy-1-naphtaldéhyde

### Exemple 33 : (Z)-α-[2-(5-Ethyl-2-furyl)-1H-imidazol-4-yl]-N-tertbutylnitrone

On procède comme dans l'exemple 1, des stades A à D, en utilisant comme substrat au stade A, le 5-éthylfurfural

### Exemple 34 : (Z)-α-[2-(2,5-Diméthyl-1-phényl-1H-pyrrole-3-yl)-1H-imidazol-4-yl]-N-tertbutylnitrone

On procède comme dans l'exemple 1, des stades A à D, en utilisant comme substrat au stade A, le 2,5-diméthyl-1-phénylpyrrole-3-carbaldéhyde.

### Exemple 35 : (Z)-α-[2-(4-Biphényl)-1H-imidazol-4-yl]-N-tertbutylnitrone

On procède comme dans l'exemple 1, des stades A à D, en utilisant comme substrat au stade A, le 4-phénylbenzaldéhyde

### Exemple 36 : (Z)-α-[2-(Benzo[b]furan-2-yl)-1H-imidazol-4-y]-N-tertbutylnitrone

On procède comme dans l'exemple 1, des stades A à D, en utilisant comme substrat au stade A, le benzo[b]furan-2-carbaldéhyde

### Exemple 37 : (Z)-α-[2-(4-Acétoxy-3-méthoxyphényl)-1H-imidazol-4-yl]-N-tertbutylnitrone

On procède comme dans l'exemple 1, des stades A à D, en utilisant comme substrat au stade A, le 4-acétoxy-3-méthoxybenzaldéhyde.

### Exemple 38 : (Z)-α-[2-(3-Quinolinyl)- 1H-imidazol-4-yl]-N-tertbutylnitrone

On procède comme dans l'exemple 1, des stades A à D en utilisant comme substrat au stade A, la quinoline-3-carbaldéhyde

### Exemple 39 : (Z)-α-[2-(2,3-Dihydro-1H-indol-3-yl)-1H-imidazol-4-yl]-N-tertbutylnitrone

On procède comme dans l'exemple 1, des stades A à D, en utilisant comme substrat au stade A, l'indoline-3-carbaldéhyde

### Exemple 40 : (Z)-α-(4-Phényl-1H-imidazol-2-yl)-N-tertbutylnitrone

### Stade A2 : 4-Phényl-1-triphénylméthyl-1H-imidazole

A une solution de 6,9 mmol de 4-phényl-1*H*-imidazole dans 50 ml de diméthylformamide et 10 ml de triéthylamine sous atmosphère d'argon, sont ajoutés 7,6 mmol de chlorure de triphénylméthyle dans 150 ml de diméthylformamide La solution est agitée 4 heures à température ambiante. Apres addition d'eau, le précipité obtenu est trituré dans l'éther éthylique, filtré puis séché permettant d'isoler le produit obtenu.

### Stade B2 : 2-Formyl-4-phényl-1-triphénylméthyl-1H-imidazole

25,7 mmol du produit obtenu dans le stade A2 sont agités à 0°C dans 250 ml de tétrahydrofurane anhydre, sous atmosphère d'argon, puis 25 ml de n-butyl-lithium (1,6 M dans l'hexane) sont additionnés lentement Après 2 heures de réaction, 8 ml de diméthylformamide sont ajoutés Après 2 heures d'agitation à température ambiante, 250 ml d'eau sont additionnés, puis le tétrahydrofurane est évaporé sous pression réduite. Une extraction de la phase aqueuse par du dichlorométhane, suivie d'une concentration, après séchage, de la phase organique, permet d'obtenir une huile qui cristallise dans l'éther éthylique, correspondant au produit attendu

### Stade C2 : (Z)-α-(4-Phényl-1-triphénylméthyl-1H-imidazol-2-yl)-N-tertbutylnitrone

On procède comme dans le stade D de l'exemple 1, en utilisant comme substrat, le produit obtenu au stade B2

### Stade D2 : (Z)-α-(4-Phényl-1H-imidazol-2-yl)-N-tertbutylnitrone

11,5 mol du produit obtenu dans le stade C2 sont chauffés au reflux, dans 60 ml d'une solution d'éthanol contenant 5 ml d'acide acétique Après 5 heures de réaction, le milieu réactionnel est concentré sous pression réduite, repris avec du dichlorométhane, lavé avec une solution de bicarbonate de sodium à 5 %, puis avec de l'eau. Après séchage sur sulfate de magnésium de la phase organique et concentration sous pression réduite, l'huile résiduelle obtenue est triturée dans l'éther éthylique, permettant d'isoler sous forme cristalline, le produit attendu
*Point de fusion : 166°C*

| *Microanalyse élémentaire :* | | | |
|---|---|---|---|
| | *C* | *H* | *N* |
| *% Calculé* | *69,11* | *7,04* | *17,27* |
| *% Trouvé* | *69,07* | *7,02* | *17,15* |

### Exemple 41 : (Z)-α-(1-Benzyl-2-phényl-1H-imidazol-4-yl)-N-tertbutylnitrone

### Stade E2 : 1-Benzyl-2-phényl-4-(trifluorométhyl)-1H-imidazole

On procède comme dans le stade E de l'exemple 8 en utilisant comme réactif le chlorure de benzyle.

### Stade F2 : (Z)-α-(1-Benzyl-2-phényl-1H-imidazol-4-yl)-N-tertbutylnitrone

On procède comme dans le stade F de l'exemple 8 en utilisant comme substrat le composé obtenu dans le stade E2

### Exemple 42 : (Z)-α-[2-(2,3-Dihydro-1,4-benzodioxin-6-yl)-1H-imidazol-4-yl]-N-tertbutylnitrone

On procède comme dans l'exemple 1, des stades A à D, en utilisant comme substrat au stade A, le 2,3-dihydro-1,4-benzodioxine-6-carbaldéhyde

### Exemple 43 : (Z)-α-[2-(2,2-Difluoro-1,3-benzodioxol-5-yl)-1H-imidazol-4-yl]-N-tertbutylnitrone

On procède comme dans l'exemple 1, des stades A à D, en utilisant comme substrat au stade A, le 2,2-difluoro-1,3-benzodioxole-5-carbaldéhyde

### Etude pharmacologique des composés de l'invention

### Exemple 44 : Test de cytotoxicité à l'acide L-homocystéique

Ce test permet d'évaluer in vitro les propriétés neuroprotectrices des composés testés, en déterminant leur capacité à s'opposer a la cytotoxicité provoquée par une exposition des cellules murines hippocampiques HT 22 à l'acide L-homocystéique (*Neuron. 2,* 1989, *1547*-1558).

Des cellules hippocampiques murines HT 22 en culture sont préincubées pendant 24 heures en présence de sept concentrations (5, 10, 25, 50, 75, 100 et 200 µM) de l'agent "antioxydant" étudié Les cultures cellulaires sont ensuite exposées pendant 24 heures à 2 mM d'acide L-homocystéique en présence ou en absence d'agents anti-oxydants. La cytotoxicité est évaluée par la méthode de réduction du bromure de 3-(4,5-diméthylthiazol-2-yl)-2,5-diphényl-tétrazolium (*Immunol. Methods*, 1983, 65,55-63). Les résultats sont exprimés par la PC 50, concentration représentant 50 % de protection par rapport à la cytotoxicité mesurée dans les cultures cellulaires en absence d'agents anti-oxydants. Lors de ce test, le composé de l'exemple 1 présente une PC 50 de 88 µM

### Exemple 45 : Test de létalité

Ce test permet de determiner in vivo les propriétés neuroprotectrices des composés testés, en mesurant leur capacité à s'opposer à la létalité induite, chez la souris, par une administration intracerébroventriculaire de t-butylhydroperoxyde, agent oxydant capable de provoquer des neurodégénerescences de type apoptotique (*Free Radical Biol. Med*., 1993, 15, 195-202 et *Mol. Chem. Neuropathol*, 1991, 26, 95-106)

L'administration intracérébroventriculaire de t-butylhydroperoxyde (1µl d'une solution à 70 %) provoque une létalité chez la souris mâle NMRI (30-35 g). La létalité est mesurée deux heures après l'administration de t-butylhydroperoxyde et est exprimée en pourcentage de protection par rapport à la létalité chez les animaux ayant reçu le véhicule des agents "anti-oxydants" étudiés Ceux-ci sont administrés par voie intrapéritonéale 30 minutes avant l'administration de t-butylhydroperoxyde, à la dose de 150 mg/kg. Lors de ce test, le composé de l'exemple 1 fournit 80 % de protection

### Exemple 46 : Evaluation des effets des agents anti-oxydants sur la température corporelle

Tous les composés anti-oxydants décrits dans la littérature sont neuroprotecteurs à doses hypothermisantes, ce qui constitue un handicap lors de leur administration.

La température corporelle est mesurée, à l'aide d'une sonde rectale, chez des souris adultes mâles NMRI (25-30 g), 30, 60, 90 et 120 minutes après l'administration par voie intrapéritonéale, des agents anti-oxydants étudiés, à la dose de 150 mg/kg. Les résultats sont exprimés en différence de température maximale moyenne déterminée chez les animaux traités par rapport à des animaux témoins ayant reçu uniquement le véhicule (20 ml/kg) des agents anti-oxydants étudiés Lors de ce test, le composé de l'exemple 1 ne crée pas d'hypothermie associée, resultat particulièrement surprenant et intéressant pour cette catégorie de composé

### Exemple 47 : Test d'ischémie cérébrale globale transitoire chez le rat

Ce test permet d'évaluer les propriétés neuroprotectrices (anti-ischémiques) des composés testés. Il a été réalisé selon la méthode de Pulsinelli et Brierley (*Stroke*, 10, 1979, 267-272).

Des rats Wistar adultes mâles, de 280-320 g, sont anesthésiés au pentobarbital (60 mg/kg i p.). Les 2 artères vertébrales sont définitivement occluses par cautérisation, et des clamps atraumatiques sont placés autour des 2 carotides communes Le lendemain, l'ischémie globale transitoire est pratiquée chez le rat éveillé en serrant les clamps carotidiens pendant 10 min Le clampage carotidien résulte en une perte du réflexe de retournement des animaux, dans la minute qui suit le clampage et pendant toute la durée de l'ischémie. Les produits étudiés sont administrés par voie intrapéritonéale 30 min avant le clampage des carotides, en utilisant le solvant approprié (2 ml/kg). Les animaux témoins reçoivent uniquement le solvant des produits étudiés Sept jours après l'ischémie, les animaux sont sacrifiés et les cerveaux sont prélevés et stockés à -30°C. Des coupes frontales de cerveau (7 µM) sont réalisées en congélation et colorées par éosine-hématoxyline. La neurodégénérescence des neurones hippocampiques (couche CA1), induite par l'ischémie, est mesurée par comptage neuronal sous microscopie optique Le paramètre mesuré est le nombre de neurones viables, exprimé en % de celui déterminé chez les animaux témoins non ischémiés.

A la dose de 100 mg/kg i.p, le *tert*-butylphénylnitrone diminue d'un facteur 2,3 la mort neuronale hippocampique chez les animaux ischémiés.
A la dose de 75 mg/kg i.p., le composé de l'exemple 14 diminue d'un facteur 3 la mort neuronale hippocampique chez les animaux ischémiés.

### Exemple 48 : Composition pharmaceutique

| Formule de préparation pour 1000 comprimés dosés à 20 mg | |
|---|---|
| Composé de l'exemple 1 | 20 g |
| Hydroxypropylcellulose | 3 g |
| Polivinylpyrrolidone | 3 g |
| Amidon de blé | 10 g |
| Lactose | 100 g |
| Stéarate de Magnésium | 3 g |

## Revendications

1. Composés de formule (I) : 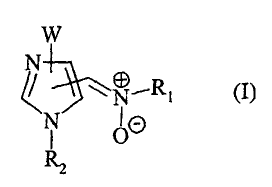 dans laquelle :
**W** représente un groupement aryle, arylalkyle (C₁-C₆) linéaire ou ramifié, hétéroaryle ou hétéroarylalkyle (C₁-C₆) linéaire ou ramifié,
**R**_{**1**} représente un groupement alkyle (C₁-C₆) linéaire ou ramifié, alkényle (C₂-C₆) linéaire ou ramifié, alkynyle (C₂-C₆) linéaire ou ramifié, aryle, cycloalkyle, arylalkyle (C₁-C₆) linéaire ou ramifié, ou cycloalkylalkyle (C₁-C₆) linéaire ou ramifié,
**R**_{**2**} représente un atome d'hydrogène, ou un groupement alkyle (C₁-C₆) linéaire ou ramifié,
leurs isomères optiques, ainsi que leurs sels d'addition à un acide ou à une base pharmaceutiquement acceptable,
étant entendu que :
- par cycloalkyle, on entend un groupement mono ou bicyclique, saturé ou insaturé mais non aromatique, comportant de 3 à 10 atomes de carbone, chacun de ces groupements pouvant être substitué de façon identique ou différente, par un ou plusieurs atomes d'halogéne, groupements hydroxy, alkyle (C₁-C₆) linéaire ou ramifié, alkoxy (C₁-C₆) linéaire ou ramifié, acyle (C₁-C₆) linéaire ou ramifié, amino (lui-même éventuellement substitué par un ou deux groupements, identiques ou différents, alkyle (C₁-C₆) linéaire ou ramifié), alkoxycarbonyle (C₁-C₆) linéaire ou ramifié, mercapto, alkylthio (C₁-C₆) linéaire ou ramifié,
- par groupement aryle, on entend un groupement phényle, naphtyle, indényle, tétrahydronaphtyle, dihydronaphtyle, dihydroindényle, chacun de ces groupements étant éventuellement substitué de façon identique ou différente, par un ou plusieurs groupements Q, tel que Q représente un atome d'halogène, un groupement hydroxy, cyano, nitro, alkyle (C₁-C₆) linéaire ou ramifié, aryle, hétéroaryle (étant entendu que dans le cas où Q représente un groupement aryle ou hétéroaryle, alors ledit groupement aryle ou ledit groupement hétéroaryle ne peut pas être substitué par un autre groupement aryle ou hétéroaryle), alkoxy (C₁-C₆) linéaire ou ramifié, trihalogénoalkyle (C₁-C₆) linéaire ou ramifié, trihalogénoalkoxy (C₁-C₆) linéaire ou ramifié, acyle (C₁-C₆) linéaire ou ramifié, carboxy, alkoxycarbonyle (C₁-C₆) linéaire ou ramifié, alkylcarbonyloxy (C₁-C₆) linéaire ou ramifié, amino (substitué éventuellement par un ou deux groupements, identiques ou différents, choisis parmi alkyle (C₁-C₆) linéaire ou ramifié, hydroxyalkyle (C₁-C₆) linéaire ou ramifié, alkoxyalkyle (C₁-C₆) linéaire ou ramifié, aminoalkyle (C₁-C₆) linéaire ou ramifié, et mono ou dialkylaminoalkyle (C₁-C₆) linéaire ou ramifié), mercapto, alkylthio (C₁-C₆) linéaire ou ramifié, aminocarbonyle (la partie amino étant éventuellement substituée par un ou deux groupements identiques ou différents, alkyle (C₁-C₆) linéaire ou ramifié), alkylcarbonylamino (C₁-C₆) linéaire ou ramifié, hydroxysulfonyle, alkylsulfonyle (C₁-C₆) linéaire ou ramifié, ou arylsulfonyle,
- et par hétéroaryle, on entend un groupement aryle, mono ou bicyclique, de 5 à 12 chaînons contenant un, deux ou trois hétéroatomes, identiques ou différents, choisis parmi oxygène, azote ou soufre, étant entendu que l'hétéroaryle peut être éventuellement substitué, de façon identique ou différente, par un ou plusieurs groupements Q tels que définis précédemment, et que dans le cas où l'hétéroaryle est bicyclique, alors un des cycles peut être partiellement hydrogéné.

2. Composés de formule (I) selon la revendication 1 **caractérisés en ce que** W est lié en position 2 sur le cycle imidazolique, leurs isomères optiques ainsi que leurs sels d'addition à un acide ou à une base pharmaceutiquement acceptable.

3. Composés de formule (I) selon la revendication 1 **caractérisés en ce que** le groupement nitrone substitué 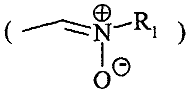 est lié en position 2 sur le cycle imidazolique, leurs isomères optiques ainsi que leurs sels d'addition à un acide ou à une base pharmaceutiquement acceptable.

4. Composés de formule (I) selon la revendication 1 **caractérisés en ce que** R₁ représente un groupement alkyle (C₁-C₆) linéaire ou ramifié, leurs isomères optiques ainsi que leurs sels d'addition à un acide ou à une base pharmaceutiquement acceptable.

5. Composés de formule (I) selon l'une des revendications 1 ou 4 **caractérisés en ce que** R₁ représente un groupement tert-butyle, leurs isomères optiques ainsi que leurs sels d'addition à un acide ou à une base pharmaceutiquement acceptable.

6. Composés de formule (I) selon la revendication 1 **caractérisés en ce que** W représente un groupement aryle ou un groupement hétéroaryle, leurs isomères optiques ainsi que leurs sels d'addition à un acide ou à une base pharmaceutiquement acceptable.

7. Composés de formule (I) selon l'une des revendications 1 ou 6 **caractérisés en ce que** W représente un groupement phényle substitué ou non, naphtyle substitué ou non, pyridinyle substitué ou non, furyle substitué ou non, thiényle substitué ou non, pyrrolyle substitué ou non, imidazolyle substitué ou non, thiazolyle substitué ou non, quinolyle substitué ou non, ou indolyle substitué ou non, leurs isomères optiques ainsi que leurs sel d'addition à un acide ou à une base pharmaceutiquement acceptable.

8. Composé de formule (I) selon la revendication 1 qui est le (Z)-α-(2-phényl-1*H*-imidazol-4-yl)-N-*tert*butylnitrone.

9. Procédé de préparation des composés de formule (I) **caractérisé en ce que** l'on utilise comme produit de départ un composé de formule (II) : 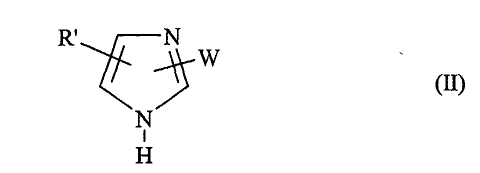 dans laquelle W est tel que défini dans la formule (I) et R' représente un atome d'hydrogène ou un groupement trifluorométhyle,
• composé de formule (II), dans le cas où W est lié en position 4 sur le cycle imidazolique et R' représente un atome d'hydrogène, que l'on traite par de l'ortho-formiate d'éthyle en présence d'acide para-toluène sulfonique, pour conduire aux composés de formule (III): 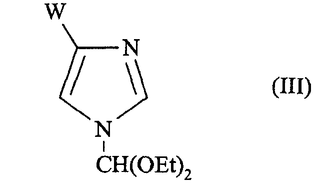 dans laquelle W est tel que défini dans la formule (I),
composés de formule (III), que l'on traite en présence d'une base forte par du diméthylformamide, suivie d'une hydrolyse acide, pour conduire aux composés de formule (IV): 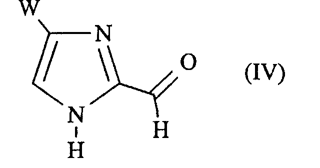 dans laquelle W est tel que défini dans la formule (I),
composé de formule (IV)
* que l'on traite par une hydroxylamine substituée de formule (V) :
HO-NH-R₁ (V)
dans laquelle R₁ est tel que défini dans la formule (I),
pour conduire aux composés de formule (I/a), cas paiticulier des composés de formule (I) : 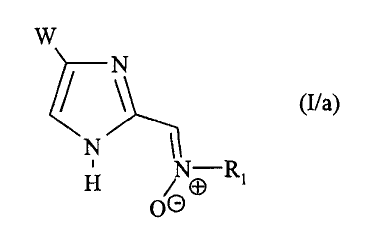 dans laquelle W et R₁ sont tels que définis dans la formule (I),
* ou que l'on traite, si on le souhaite, avec un réactif électrophile selon des conditions classiques de synthèse organique, pour conduire aux composés de formules (IV A) et (IV B) : 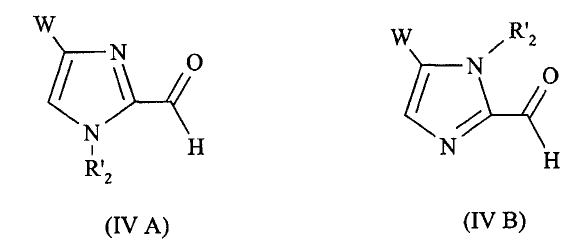 dans lesquelles W est tel que défini dans la formule (I) et R'₂ a la même définition que R₂ dans la formule (I), excepté que R'₂ ne peut pas représenter un atome d'hydrogène,
composés de formules (IV A) et (IV B) que l'on sépare et/ou que l'on purifie si on le souhaite selon des méthodes classiques de séparation et de purification, puis que l'on soumet à l'action d'un composé de formule (V) tel que défini précédemment, pour conduire, respectivement, aux composés de formules (I/b) et (I/c), cas particuliers des composés de formule (I) : 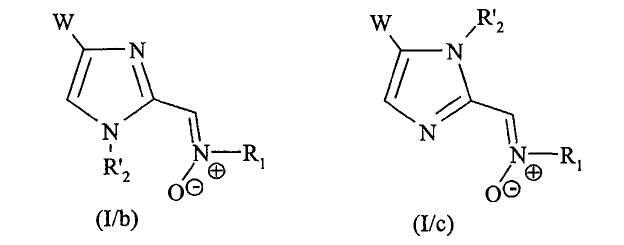 dans lesquelles R₁, R'₂ et W sont tels que définis précédemment,
• ou composé de formule (II), dans le cas où W est lié en position 2 sur le cycle imidazolique et R' représente un groupement trifluorométhyle : 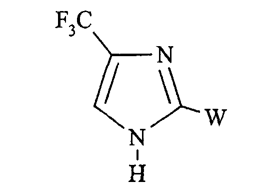 que l'on traite soit par de l'hydroxyde d'ammonium, suivi d'une hydrolyse, soit par de la soude suivi d'une étape d'addition de N-méthoxy-N-méthylamine puis d'une réaction de réduction,
pour conduire aux composés de formule (VI) : 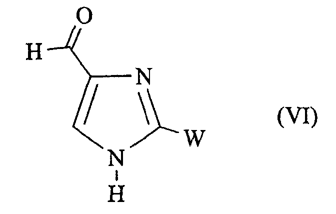 dans laquelle W est tel que défini dans la formule (I),
composés de formule (VI) que l'on soumet à l'action d'un composé de formule (V) tel que défini précédemment, pour conduire aux composés de formule (I/d), cas particulier des composés de formule (I) : 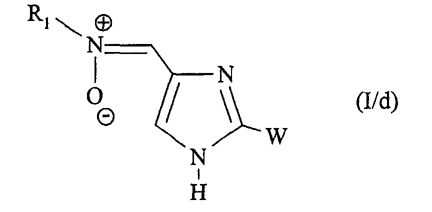 dans laquelle R₁ et W sont tels que définis dans la formule (I),
ou composés de formule (VI), que l'on traite, si on le souhaite, avec un réactif électrophile, selon des conditions classiques de synthèse organique, pour conduire aux composés de formules (VI A) et (VI B) : 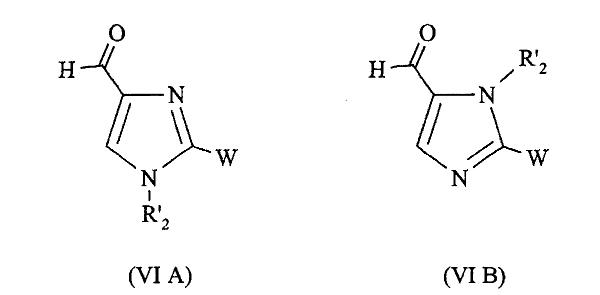 dans lesquelles W et R'₂ sont tels que définis précédemment,
composés de formules (VI A) et (VI B) que l'on sépare et/ou que l'on purifie selon des méthodes classiques de séparation et de purification, puis que l'on soumet à l'action d'un composé de formule (V), pour conduire, respectivement, aux composés de formules (I/e) et (I/f), cas particulier des composés de formule (I) : 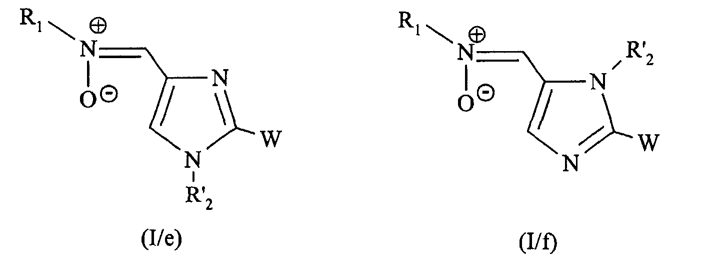 dans lesquelles R₁, R'₂ et W sont tels que définis précédemment,
les composés (I/a) à (I/f) formant l'ensemble des composés de l'invention, que l'on purifie, le cas échéant, selon une technique classique de purification, qui peuvent, si on le désire, être séparés en leurs différents isomères optiques selon une technique classique de séparation, et que l'on transforme, le cas échéant, en leurs sels d'addition à un acide ou à une base pharmaceutiquement acceptable.

10. Compositions pharmaceutiques contenant comme principe actif au moins un composé de formule (I) selon l'une quelconque des revendications 1 à 8, seul ou en combinaison avec un ou plusieurs excipients ou véhicules inertes, non toxiques, pharmaceutiquement acceptables.

11. Compositions pharmaceutiques selon la revendication 10 contenant au moins un principe actif selon l'une quelconque des revendications 1 à 8 utiles en tant qu'agent antioxydant.

12. Compositions pharmaceutiques selon la revendication 10 contenant au moins un principe actif selon l'une quelconque des revendications 1 à 8 utiles en tant qu'agent antioxydant dans le traitement des troubles cognitifs associés au vieillissement cérébral ou aux maladies neurodégénératives, et/ou dans le traitement des maladies neurodénégératives aiguës et progressives.

## Claims

1. Compounds of formula (I): 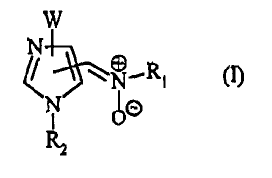 wherein:
**W** represents an aryl group, an aryl-(C₁-C₆)alkyl group in which the alkyl moiety may be linear or branched, a heteroaryl group or a heteroaryl-(C₁-C₆)alkyl group in which the alkyl moiety may be linear or branched,
**R**_{**1**} represents a linear or branched (C₁-C₆)alkyl group, a linear or branched (C₂-C₆)alkenyl group, a linear or branched (C₂-C₆)alkynyl group, an aryl group, a cycloalkyl group, an aryl-(C₁-C₆)alkyl group in which the alkyl moiety may be linear or branched, or a cycloalkyl-(C₁-C₆)alkyl group in which the alkyl moiety may be linear or branched,
**R**_{**2**} represents a hydrogen atom or a linear or breached (C₁-C₆)alkyl group,
their optical isomers, and also addition salts thereof with a pharmaceutically acceptable acid or base,
wherein
- a "cycloalkyl" group is to be understood as meaning a mono- or bi-cyclic group that is saturated or unsaturated but is not aromatic and that contains from 3 to 10 carbon atoms, it being possible for each of those groups to be substituted by one or more identical or different substituents selected from halogen atoms, hydroxy groups, linear or branched (C₁-C₆)alkyl groups, linear or branched (C₁-C₆)alkoxy groups, linear or branched (C₁-C₆)acyl groups, amino groups (wherein amino is itself optionally substituted by one or two identical or different linear or branched (C₁-C₆)alkyl groups), linear or branched (C₁-C₆)alkoxycarbonyl groups, mercapto groups and linear or branched (C₁-C₆)alkylthio groups,
- a "heterocycloalkyl" group is to be understood as meaning a cycloalkyl group in which one, two or three carbon atoms have been replaced by identical or different hetero atoms selected from nitrogen, oxygen and sulphur,
- an "aryl" group is to be understood as meaning a phenyl, naphthyl, indenyl, tetrahydronaphthyl, dihydronaphthyl or dihydroindenyl group, each of which is optionally substituted by one or more identical or different groups Q, Q representing a halogen atom or one of the following groups: hydroxy, cyano, nitro, linear or branched (C₁-C₆)alkyl, aryl, heteroaryl (it being understood that when Q represents an aryl or heteroaryl group, then the said aryl group or the said heteroaryl group may not be substituted by another aryl or heteroaryl group), linear or branched (C₁-C₆)alkoxy, linear or branched trihalo-(C₁-C₆)alkyl, linear or branched trihalo-(C₁-C₆)alkoxy, linear or branched (C₁-C₆)acyl, carboxy, linear or branched (C₁-C₆)alkoxycarbonyl, linear or branched (C₁-C₆)alkylcarbonyloxy, amino (optionally substituted by one or two identical or different groups selected from linear or branched (C₁-C₆)alkyl, linear or branched hydroxy-(C₁-C₆)alkyl, linear or branched alkoxy-(C₁-C₆)alkyl, amino-(C₁-C₆)-alkyl in which the alkyl moiety may be linear or branched, and mono- or di-alkylaminoalkyl in which alkyl may be linear or branched and contains from 1 to 6 carbon atoms), mercapto, linear or branched (C₁-C₆)alkylthio, aminocarbonyl (the amino moiety optionally being substituted by one or two identical or different linear or branched (C₁-C₆)alkyl groups), linear or branched (C₁-C₆)alkylcarbonylamino, hydroxysulphonyl, linear or branched (C₁-C₆)alkylsulphonyl, or arylsulphonyl, and
- a "heteroaryl" group is to be understood as meaning a mono- or bi-cyclic aryl group having from 5 to 12 ring members containing one, two or three identical or different hetero atoms selected from oxygen, nitrogen and sulphur, it being understood that the heteroaryl may optionally be substituted by one or more identical or different groups Q as defined hereinbefore, and that when the heteroaryl is bicyclic one of the rings may be partially hydrogenated.

2. Compounds of formula (I) according to claim 1, **characterised in that** W is attached in the 2 position of the imidazole ring, their optical isomers, and also addition salts thereof with a pharmaceutically acceptable acid or base.

3. Compounds of formula (I) according to claim 1, **characterised in that** the substituted nitrone group 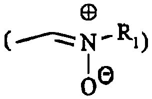 is attached in the 2 position of the imidazole ring, their optical isomers, and also addition salts thereof with a pharmaceutically acceptable acid or base.

4. Compounds of formula (I) according to claim 1, **characterised in that** R₁ represents a linear or branched (C₁-C₆)alkyl group, their optical isomers, and also addition salts thereof with a pharmaceutically acceptable acid or base.

5. Compounds of formula (I) according to either claim 1 or claim 4, **characterised in that** R₁ represents a *tert*-butyl group, their optical isomers, and also addition salts thereof with a pharmaceutically acceptable acid or base.

6. Compounds of formula (I) according to claim 1, **characterised in that** W represents an aryl group or a heteroaryl group, their optical isomers, and also addition salts thereof with a pharmaceutically acceptable acid or base.

7. Compounds of formula (I) according to either claim 1 or claim 6, **characterised in that** W represents an unsubstituted or substituted phenyl group, an unsubstituted or substituted naphthyl group, an unsubstituted or substituted pyridyl group, an unsubstituted or substituted furyl group, an unsubstituted or substituted thienyl group, an unsubstituted or substituted pyrrolyl group, an unsubstituted or substituted imidazolyl group, an unsubstituted or substituted thiazolyl group, an unsubstituted or substituted quinolyl group or an unsubstituted or substituted indolyl group, their optical isomers, and also addition salts thereof with a pharmaceutically acceptable acid or base.

8. Compound of formula (I) according to claim 1 that is (Z)-α-(2-phenyl-1*H*-imidazol-4-yl)-N-*tert*-butylnitrone.

9. Process for the preparation of compounds of formula (I), **characterised in that** there is used as starting material a compound of formula (II): 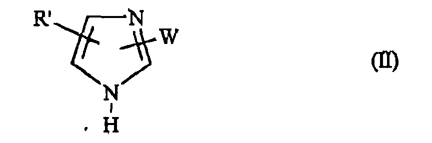 wherein W is as defined for formula (I) and R' represents a hydrogen atom or a trifluoromethyl group,
• which compound of formula (II), when W is attached in the 4 position of the imidazole ring and R' represents a hydrogen atom, is treated with ethyl orthoformate in the presence of para-toluenesulphonic acid to yield compounds of formula (III) : 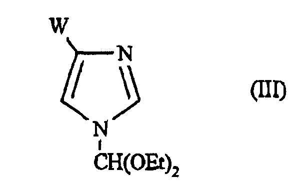 wherein W is as defined for formula (I),
which compounds of formula (III) are treated in the presence of a strong base with dimethylformamide, this being followed by acid hydrolysis, to yield compounds of formula (TV): 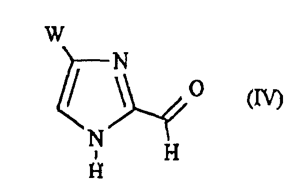 wherein W is as defined for formula (I),
which compound of formula (IV)
* is treated with a substituted hydroxylamine of formula (V):
HO-NH-R₁ (V)
wherein R₁ is as defined for formula (I),
to yield the compounds of formula (I/a), a particular case of the compounds of formula (I): 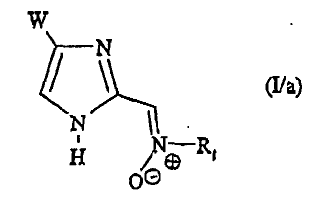 whexein Wand R₁ are as defined for formula (I),
* or is treated, if desired, with an electrophilic reagent in accordance with conventional conditions of organic synthesis to yield the compounds of formulae (IV A) and (IV B): 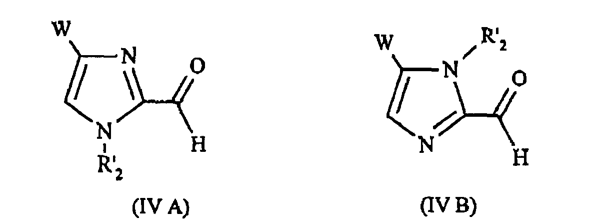 wherein W is as defined for formula (I) and R'₂ has the same meanings as R₂ in formula (I), except that R'₂ may not represent a hydrogen atom,
which compounds of formulae (IV A) and (IV B) are separated and/or purified, if desired, according to conventional methods of separation and purification, and are then subjected to the action of a compound of formula (V) as defined hereinbefore to yield, respectively, the compounds of formulae (I/b) and (I/c), particular cases of the compounds of formula (I): 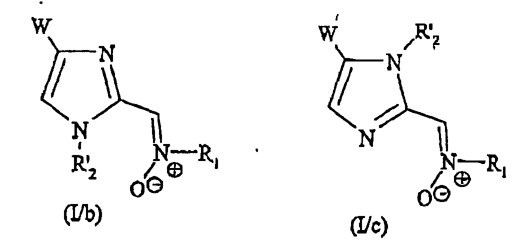 wherein R₁, R'₂ and W are as defined hereinbefore,
• or which compound of formula (II), when W is attached in the 2 position of the imidazole ring and R' represents a trifluoromethyl group: 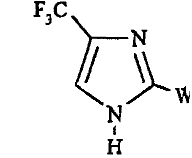 is either treated with ammonium hydroxide and then subjected to hydrolysis, or is treated with sodium hydroxide and then subjected to an N-methoxy-N-methylamine addition step followed by a reduction reaction,
to yield the compounds of formula (VI): 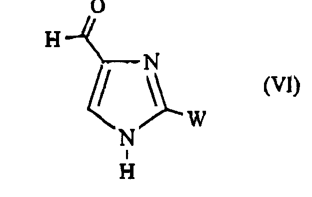 wherein W is as defined for formula (I),
which compounds of formula (VI) are subjected to the action of a compound of formula (V) as defined hereinbefore to yield the compounds of formulae (I/d), a particular case of the compounds of formula (I): 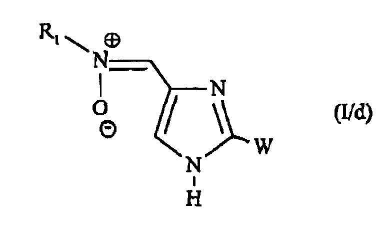 wherein R₁ and W are as defined for formula (I),
or which compounds of formula (VI) are, if desired, treated with an electrophilic reagent, in accordance with conventional conditions of organic synthesis, to yield the compounds of formulae (VI A) and (VI B): 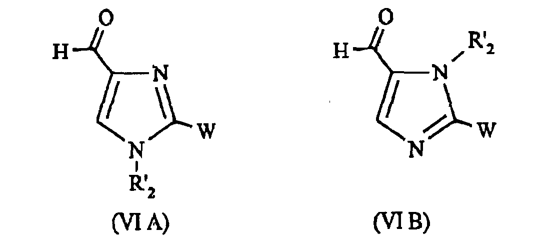 wherein W and R'₂ are as defined hereinbefore,
which compounds of formulae (VI A) and (VI B) arc separated and/or purified according to conventional methods of separation and purification, then subjected to the action of a compound of formula (V) to yield, respectively, the compounds of formulae (I/e) and (I/f), particular cases of the compounds of formula (I): 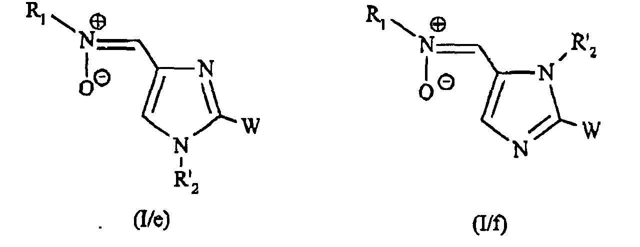 wherein R₁, R'₂ and W are as defined hereinbefore,
the compounds of formulae (I/a) to (I/f) constituting the totality of the compounds of the invention, which compounds are purified, if necessary, according to a conventional purification technique, may be separated, if desired, into their different optical isomers according to a conventional separation technique, and are converted, if desired, into addition salts with a pharmaceutically acceptable acid or base.

10. Pharmaceutical compositions comprising as active ingredient at least one compound of formula (1) according to any one of claims 1 to 8, alone or in combination with one or more pharmaceutically acceptable, inert, non-toxic excipients or carriers.

11. Pharmaceutical compositions according to claim 10 comprising at least one active ingredient according to any one of claims 1 to 8 for use as an anti-oxidation agent

12. Pharmaceutical compositions according to claim 10 comprising at least one active ingredient according to any one of claims 1 to 8 for use as an anti-oxidation agent in the treatment of cognitive disorders associated with cerebral ageing or neurodegenerative disorders, and/or in the treatment of acute and progressive neurodegenerative disorders.

## Patentansprüche

1. Verblödungen der Formel (I); 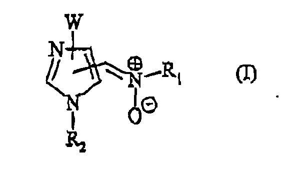 in der:
**W** eine Arylgruppe, geradkettige oder verzweigte Aryl-(C₁-C₆)-alkylgruppe, Heteroarylgruppe oder geradkettige oder verzweigte Heteroaryl-(C₁-C₆)alkylgruppe darstellt,
**R**_{**1**} eine geradkettige oder verzweigte (C₁-C₆)-Alkylgruppe, geradkettige oder verzweigte (C₂-C₆)-Alkenylgruppe, geradkettige oder verzweigte (C₂-C₆)-Alkinylgruppe, Arylgruppe. Cycloalkylgruppe, geradkettige oder verzweigte Aryl-(C₁-C₆)-alkylgruppe oder geradkettige oder verzweigte Cycloalkyl-(C₁-C₆)-alkylgruppe bedeutet,
**R**_{**2**} ein Wasserstoffatom oder eine geradkettige oder verzweigte (C₁-C₆)-Alkylgruppe darstellt,
deren optische Isomere sowie deren Additionssalze mit einer pharmazeutisch annehmbaren Säure oder Base.
mit der Maßgabe, daß:
- unter Cycloalkyl eine mono- oder bicyclische, gesättigte oder ungesättigte jedoch nicht aromatische Gruppe zu verstehen ist, die 3 bis 10 Kohlenstoffatome enthält, wobei jede dieser Gruppen in gleichartiger oder unterschiedlicher Weise substituiert sein kann durch ein oder mehrere Halogenatome, Hydroxygruppen, geradkettige oder verzweigte (C₁-C₆)-Alkylgruppen, geradkettige oder verzweigte (C₁-C₆)-Alkoxygruppen, geradkettige oder verzweigte (C₁-C₆)-Acylgruppen, Aminogruppen (die ihrerseits gegebenenfalls durch eine oder zwei gleichartige oder verschiedene, geradkettige oder verzweigte (C₁-C₆)-Alkylgruppen substituiert sein können), geradkettige oder verzweigte (C₁-C₆)-Alkoxycarbonylgruppen, Mercaptogruppen oder geradkettige oder verzweigte (C₁-C₆)-Alkylthiogruppen
- unter einer Heterocycloalkylgruppe eine Cycloalkylgruppe zu verstehen ist. in der eines, zwei oder drei Kohlenstoffatome durch ein Heteroatom ersetzt sind. welches in gleichartiger oder unterschledlicher Weise aus Stickstoff, Sauerstoffund Schwefel ausgewählt ist,
- unter einer Arylgruppe eine Phenyl-, Naphthyl-, Indenyl-, Tetrahydronaphthyl-, Dihydronaphthyl- und Dihydroindenylgruppe zu verstehen ist, wobei jede dieser Gruppen gegebenenfalls in gleichartiger oder unterschiedlicher Weise substituiert ist durch eine oder mehrere Gruppen Q, worin Q ein Halogenatom, eine Hydroxygruppe, Cyanogruppe, Nitrogruppe, geradkettige oder verzweigte (C₁-C₆)-Alkylgnappe, Arylgruppe, Heteroarylgruppe (wobei es sich versteht, daß dann, wenn Q eine Aryl- oder Heteroarylgruppe darstellt, die Arylgruppe oder die Heteroarylgruppe nicht durch eine andere Aryl- oder Heteroarylgruppe substituiert sein kann), geradkettige oder verzweigte (C₁-C₆)-Alkoxygruppe, geradkettige oder verzweigte (C₁-C₆)-Trihalogenalkylgruppe, geradkettige oder verzweigte (C₁-C₆)-Trihalogenalkoxygruppe, geradkettige oder verzweigte (C₁-C₆)-Acylgruppe, Carboxygruppe, geradkettige oder verzweigte (C₁-C₆)-Alkoxycarbonylgruppe, geradkettige oder verzweigte (C₁-C₆)-Alkylcarbonyloxygruppe, Aminogruppe (die gegebenenfalls durch ein oder zwei gleichartige oder verschiedene Gruppen ausgewählt aus geradkettigem oder verzweigtem (C₁-C₆)-Alkyl, geradkettigem oder verzweigtem (C₁-C₆)-Hydroxyalkyl, geradkettigem oder verzweigtem (C₁-C₆)-Alkoxyalkyl, geradkettigem oder verzweigtem (C₁-C₆)-Aminoalkyl und geradkettigem oder verzweigtem (C₁-C₆)-Mono- oder Dialkylaminoalkyl substituiert ist), Mercaptogruppe, geradkettige oder verzweigte (C₁-C₆)-Alkylthiogruppe. Aminocarbonylgruppe (wobei der Aminorest gegebenenfalls durch ein oder zwei gleichartige oder verschiedene, geradkettige oder verzweigte (C₁-C₆)-Alkylgruppen substituiert sein kann), geradkettige oder verzweigte (C₁-C₆)-Alkylcarbonylaminogruppe, Hydroxysulfonylgruppe, geradkettige oder verzweigte (C₁-C₆)-Alkylsulfonylgruppe oder Arylsulfonylgruppe bedeutet,
- und unter Heteroaryl eine mono- oder bicyclische Arylgruppe mit 5 bis 12 Kettengliedern zu verstehen ist, die eines, zwei oder drei gleichartige oder verschiedene Heteroatome ausgewählt aus Sauerstoff, Stickstoff oder Schwefel enthält, wobei es sich versteht, daß die Heteroarylgruppe gegebenenfalls in gleichartiger oder unterschiedlicher Weise durch eine oder mehrere Gruppen Q, wie sie oben definiert worden sind, substituiert sein kann und daß dann, wenn die Heteroarylgruppe bicyclisch ist, einer der Ringe teilweise hydriert sein kann.

2. Verbindung mit der Formel (I) nach Anspruch 1, **dadurch gekennzeichnet, daß** W in der 2-Stellung des Imidazolringes gebunden ist, deren optische Isomere sowie deren Additionssalze mit einer pharmazeutisch annehmbaren Säure oder Base.

3. Verbindungen der Formel (I) nach Anspruch 1, **dadurch gekennzeichnet, daß** die substituierte Nitrongruppe 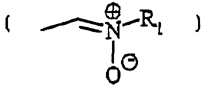 in der 2-Stellung des Imidazolringes gebunden ist, deren optische Isomere sowie deren Additionssalze mit einer pharmazeutisch annehmbaren Säure oder Base.

4. Verbindungen der Formel (I) nach Anspruch 1, **dadurch gekennzeichnet, daß** R₁ eine geradkettige oder verzweigte (C₁-C₆)-Alkylgruppe bedeutet, deren optische Isomere sowie deren Additionssalze mit einer pharmazeutisch annehmbaren Säure oder Base.

5. Verbindungen der Formel (I) nach einem der Ansprüche 1 oder 4, **dadurch gekennzeichnet, daß** R₁ eine tert.-Butylgruppe bedeutet, deren optische Isomere sowie deren Additionssalze mit einer pharmazeutisch annehmbaren Säure oder Base.

6. Verbindungen der Formel (I) nach Anspruch 1, **dadurch gekennzeichnet. daß** W eine Arylgruppe oder eine Heteroarylgruppe bedeutet, deren optische Isomere sowie deren Additionssalze mit einer pharmazeutisch annehmbaren Säure oder Base.

7. Verbindungen der Formel (I) nach einem der Ansprüche 1 oder 6, **dadurch gekennzeichnet, daß** W eine gegebenenfalls substituierte Phenylgruppe, gegebenenfalls substituierte Naphthylgruppe, gegebenenfalls substituierte Pyridinylgruppe. gegebenenfalls substituierte Furylgruppe, gegebenenfalls substituierte Thienylgruppe, gegebenenfalls substituierte Pyrrolygruppe, gegebenenfalls substituierte Imidazolylgruppe, gegebenenfalls substituierte Thiazolylgruppe, gegebenenfalls substituierte Chinolylgruppe oder gegebenenfalls substituierte Indolylgruppe bedeutet, deren optische Isomere sowie deren Additionssalze mit einer pharmazeutisch annehmbaren Säure oder Base.

8. Verbindungen der Formel (I) nach Anspruch 1, nämlich (Z)-α-(2-Phenyl-1*H*imidazol-4-yl)-N-*tert*.-butylnitron.

9. Verfahren zur Herstellung der Verbindungen der Formel (I), **dadurch gekennzeichnet, daß** man als Ausgangaprodukt eine Verbindung der Formel (II) verwendet: 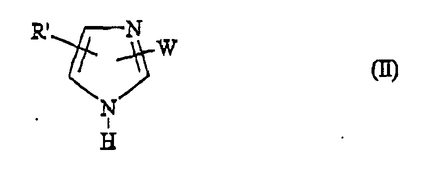 in der W die bezüglich der Formet (I) angegebenen Bedeutungen besitzt und R' ein Wasserstoffatom oder eine Trifluormethylgruppe bedeutet,
• welche Verbindung der Formel (II), in dem Fall, da W in der 4-Stellung an den Imidazolring gebunden ist und R' ein Wasserstoffatom bedeutet, man mit Orthoameisensäureethylester in Gegenwart von p-Toluolsulfonsäure behandelt zur Bildung der Verbindungen der Formel (III): 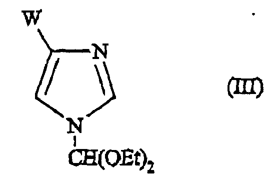 in der W die bezüglich der Formel (I) angegebenen Bedeutungen besitzt,
welche Verbindungen der Formel (III) man in Gegenwart einer starken Base mit Dimethylformamid behandelt, gefolgt von einer sauren Hydrolyse zur Bildung der Verbindungen der Formel (IV): 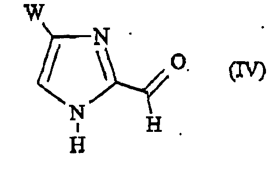 In der W die bezüglich der Formel (I) angegebenen Bedeutungen besitzt. welche Verbindung der Formel (IV)
* man mit einem substituierten Hydroxylamin der Formel (V):
HO-NH-R₁ (V)
behandelt,
in der R₁ die bezüglich der Formel (I) angegebenen Bedeutungen besitzt, zur Bildung der Verbindungen der Formel (I/a), einem Sonderfall der Verbindungen der Formel (I): 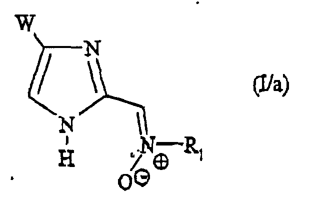 in der W und R₁ die bezüglich der Formel (I) angegebenen Bedeutungen besitzen.
* oder welche man gewünschtenfalls mit einem elektrophilen Reagenz unter den klassischen Bedingungen der organischen Synthese behandelt, zur Bildung der Verbindungen der Formeln (IV A) und (IV B): 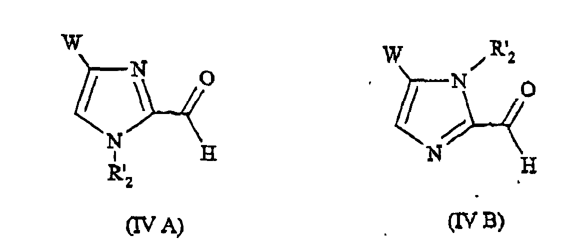 in denen W die bezüglich der Formel (I) angegebenen Bedeutungen besitzt und R'₂ die für R₂ bezüglich der Formel (I) angegebenen Bedeutungen besitzt mit der Ausnahme, daß R'₂ kein Wasserstoffatom bedeuten kann,
welche Verbindungen der Formeln (IV A) und (IV B) man gewünschtenfalls trennt und/oder reinigt unter Anwendung klassischer Methoden der Trennung und der Reinigung und welche man dann der Einwirkung einer Verbindung der oben definierten Formel (V) unterwirft zur Bildung der Verbindungen der Formein (I/b) beziehungsweise (I/c), Sonderfällen der Verbindungen der Formel (I): 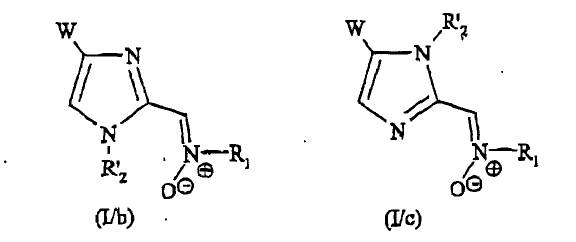 worin R₁, R'₂ und W die oben angegebenen Bedeutungen besitzen,
• oder welche Verbindung der Formel (II) man dann, wenn W in der 2-Stellung an den Imidazoiring gebunden ist und R' eine Trifluormethylgruppe bedeutet: 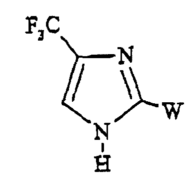 entweder mit Ammoniumhydroxyd gefolgt von einer Hydrolyse oder mit Natriumhydroxyd gefolgt von einer Stufe der Addition von N-Methoxy-N-methylamin und dann mit einer Reduktionsreaktion behandelt,
zur Bildung der Verbindungen der Formel (VI): 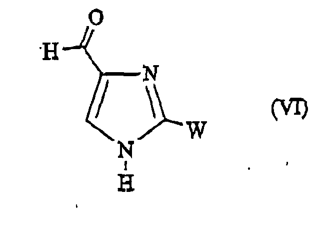 in der W die bezüglich der Formel (I) angegebenen Bedeutungen besitzt,
welche Verbindungen der Formel (VI) man der Einwirkung einer Verbindung der Formel (V), wie sie oben definiert worden ist, unterwirft zur Bildung der Verbindungen der Formel (l/d), ein Sonderfall der Verbindungen der Formel (I): 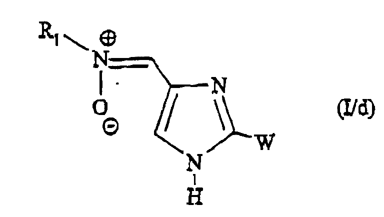 in der R₁ und W die bezüglich der Formel (I) angegebenen Bedeutungen besitzen.
oder welche Verbindungen der Formel (VI) man gewünschtenfalls mit einem elektrophilen Reagenz unter Anwendung klassischer Bedingungen der organischen Synthese behandelt zur Bildung der Verbindungen der Formeln (VI A) und (VI B): 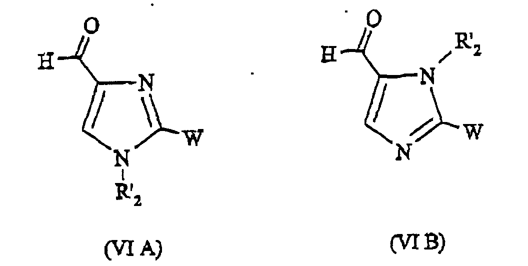 in denen W und R'₂ die oben angegebenen Bedeutungen besitzen.
welche Verbindungen der Formeln (VI A) und (VI B) man unter Anwendung klassischer Methoden der Trennung und der Reinigung trennt und/oder reinigt und dann der Einwirkung einer Verbindung der Formel (V) unterwirft, zur Bildung der Verbindungen der Formeln (I/e) beziehungsweise (T/f), Sonderfällen der Verbindungen der Formel (I): 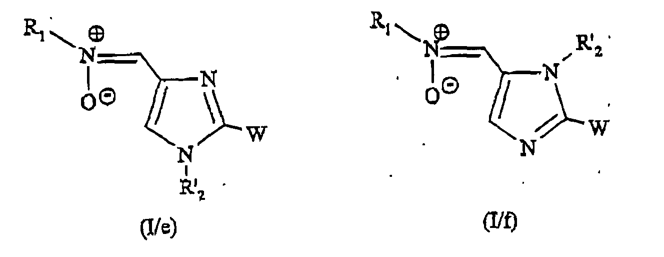 in denen R₁, R'₂ und W die oben angegebenen Bedeutungen besitzen,
wobei die Verbindungen (I/a) bis (I/f) die Gesamtheit der erfindungsgemäßen Verbindungen bilden, welche man gegebenenfalls mit Hilfe einer klassischen Reinigungsmethode reinigt, welche gewünschtenfalls mit Hilfe einer klassischen Trennmethode in ihre unterschiedlichen optischen Isomeren getrennt werden können und welche man gegebenenfalls in ihre Additionssalze mit einer pharmazeutisch annehmbaren Säure oder Base umwandelt.

10. Pharmazeutische Zubereitungen enthaltend als Wirkstoff mindestens eine Verbindung der Formel (I) nach einem der Ansprüche 1 bis 8 alleln oder in Kombination mit einem oder mehreren inerten, nichttoxischen, pharmazeutisch annehmbaren Trägermaterialien oder Bindemitteln.

11. Pharmazeutische Zubereitungen nach Anspruch 10 enthaltend mindestens einen Wirkstoff nach einem der Ansprüche 1 bis 8 als antioxidierendes Mittel.

12. Pharmazeutische Zubereitungen nach Anspruch 10 enthaltend mindestens einen Wirkstoff nach einem der Ansprüche 1 bis 8 als antioxidierendes Mittel bei der Behandlung von Erkenntnisstörungen, die mit dem Altern des Gehirns oder mit neurodegenerativen Erkrankungen verknüpft sind und/oder bei der Behandlung von akuten und progressiven neurodegenerativen Erkrankungen.
